(19) Europäisches Patentamt
European Patent Office
Office européen des brevets

(11) EP 0 784 058 B1

(12) **EUROPÄISCHE PATENTSCHRIFT**

(45) Veröffentlichungstag und Bekanntmachung des Hinweises auf die Patenterteilung:
**26.10.2005 Patentblatt 2005/43**

(51) Int Cl.[7]: **C07K 5/06**, C07D 207/04, C07D 211/06, A61K 38/04, A61K 31/445, A61K 31/40

(21) Anmeldenummer: **96102404.9**

(22) Anmeldetag: **18.03.1993**

(54) **Asparaginsäurederivate, ihre Herstellung und Verwendung**

Derivatives of aspartic acid, preparation and use thereof

Dérivatives d'acide aspartique et leur préparation et utilisation

(84) Benannte Vertragsstaaten:
**AT BE CH DE DK ES FR GB IT LI NL SE**

(30) Priorität: **13.04.1992 DE 4212304**

(43) Veröffentlichungstag der Anmeldung:
**16.07.1997 Patentblatt 1997/29**

(62) Dokumentnummer(n) der früheren Anmeldung(en) nach Art. 76 EPÜ:
**93104415.0 / 0 565 896**

(73) Patentinhaber: **Aventis Pharma Deutschland GmbH**
**65929 Frankfurt am Main (DE)**

(72) Erfinder:
- **Klingler, Otmar, Dr.**
  **D-63110 Rodgau (DE)**
- **Zoller, Gerhard, Dr.**
  **D-61137 Schöneck (DE)**
- **Just, Melitta, Dr.**
  **D-63225 Langen (DE)**
- **Jablonka, Bernd, Dr.**
  **D-61140 Oberursel (DE)**

(56) Entgegenhaltungen:
**EP-A- 0 372 486** **WO-A-91/05562**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 784 058 B1

## Beschreibung

Die Erfindung betrifft Asparaginsäurederivate der allgemeinen Formel I

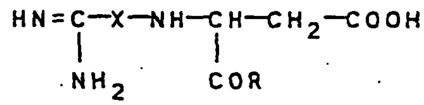 (I)

worin

X

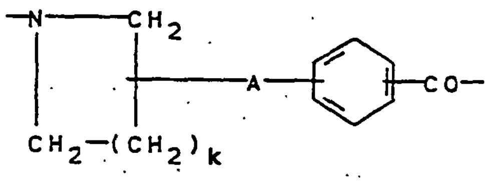 ,

A $-(CH_2)_m-$, -O- oder eine direkte Bindung,
k die Zahl 2 oder die Zahl 3,
m die Zahl 1,
R $-OR^1$,

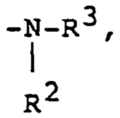

$-NH-R^4$,

$R^1$ Wasserstoff, $C_1$-$C_{28}$Alkyl, $C_3$-$C_{28}$Cycloalkyl, Phenyl wobei das $C_1$-$C_{28}$Alkyl, das-$C_3$-$C_{28}$Cycloalkyl oder das Phenyl unsubstituiert oder ein- oder mehrfach mit gleichen oder verschiedenen Resten aus der Reihe Hydroxy, Carboxy, $C_1$-$C_4$Alkoxycarbonyl, Arylmethoxycarbonyl, Carboxamido, $C_1$-$C_4$Alkylaminocarbonyl, Amino, Mercapto, $C_1$-$C_4$-Alkoxy, $C_3$-$C_8$Cycloalkyl, Imidazolyl, Indolyl, Pyrrolidinyl, Hydroxypyrrolidinyl, Halogen, unsubstituiertem oder ein oder mehrfach mit Hydroxy, $C_1$-$C_4$Alkyl, Halogen, Nitro oder Trifluormethyl, substituiertem Phenyl oder Phenoxy, substituiert ist, ein oder mehrfach durch -O-unterbrochenes $C_3$-$C_{81}$Alkyl oder ein oder mehrfach durch -O- unterbrochenes ($C_3$-$C_{81}$Alkoxy)carbonyl;
$R^2$, $R^3$ unabhängig voneinander Wasserstoff, $R^1$ oder zusammen mit dem Stickstoff, an dem sie gebunden sind, einen 5- oder 6-gliedrigen, gesättigten heterocyclischen Ring, der auch noch -O- oder $-NR^5-$ enthalten kann, oder $R^2$ Wasserstoff und $R^3$ einen Rest der Formel

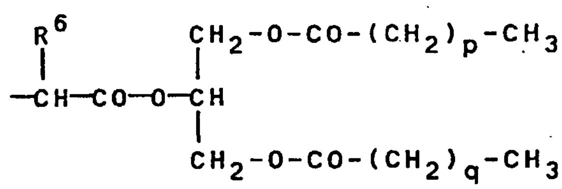

oder

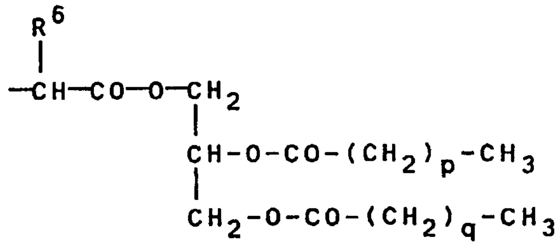

$R^4$ den Rest einer Aminosäure oder eines Dipeptids, bei dem die Peptidbindung auch zu $-NH-CH_2-$ reduziert sein kann,
$R^5$ Wasserstoff, $C_1$-$C_4$Alkyl, Phenyl, $C_1$-$C_4$Alkylphenyl, $C_1$-$C_4$Alkoxyphenyl,
$R^6$ Wasserstoff, $C_1$-$C_4$Alkyl, Phenyl, Phenyl($C_1$-$C_4$)Alkyl, p, q unabhängig voneinander eine Zahl von 1 bis 20 bedeuten, sowie deren physiologisch verträglichen Salze.

Die Erfindung betrifft auch die Verfahren zur Herstellung der Verbindungen der Formel I und ihre Verwendung als pharmazeutisch wirksame Stoffe.

Die für $R^1$, $R^2$, $R^3$ und $R^5$ stehenden Alkyl- und Alkoxyreste können geradkettig oder verzweigt sein. Dies gilt auch, wenn sie Substituenten tragen oder als Substituenten anderer Reste auftreten.

Beispiele für geeignete $C_1$-$C_{28}$Alkylreste sind: Methyl, Ethyl, Propyl, Butyl, Pentyl, Hexyl, Heptyl, Octyl, Decyl, Dodecyl, Tridecyl, Heptadecyl, Nonadecyl, Eicosyl, Docosyl, Tricosyl, Pentacosyl, Hexacosyl, Heptacosyl, Octacosyl, Isobutyl, Isopentyl, Neopentyl, Isohexyl, 3-Methylpentyl, 2,3,5-Trimethylhexyl, sec-Butyl, tert.-Butyl, tert.-Pentyl, Isohexyl. Unverzweigte $C_1$-$C_{28}$-Alkylreste sind bevorzugt.

Von den $C_3$-$C_{28}$Cycloalkylresten sind $C_3$-$C_8$Alkylreste (Cyclopropyl, Cyclobutyl, Cyclopentyl, Cyclohexyl, Cycloheptyl, Cyclooctyl), insbesondere Cyclopentyl und Cyclohexyl, bevorzugt.

Halogen kann z.B. Fluor, Chlor, Brom und Iod sein, von denen Fluor, Chlor und Brom bevorzugt sind.

Das ein- oder mehrfach durch Sauerstoff unterbrochene $C_3$-$C_{81}$Alkyl enthält bevorzugt die Gruppe

$$H_3CO\{CH_2CH_2O\}_x,$$

wobei x eine Zahl von 1 bis 40 ist.

Das ein- oder mehrfach durch Sauerstoff unterbrochene ($C_3$-$C_{81}$Alkoxy)carbonyl enthält bevorzugt die Gruppe

$$H_3CO\{CH_2CH_2O\}_xCO-$$

wobei x eine Zahl von 1 bis 40 ist.

Die $C_3$-$C_8$Cycloalkylreste können auch insbesondere durch eine Carboxygruppe substituiert sein.

Der Arylrest in dem Arylmethoxycarbonylrest kann z.B. ein α- oder β-Naphthylrest oder insbesondere ein Phenylrest sein.

Ein für $R^1$, $R^2$ oder $R^3$ auftretender Phenyl- oder Phenoxyrest oder ein als Substituent für $R^1$, $R^2$ oder $R^3$ auftretender Phenyl- oder Phenoxyrest kann z.B. ein-, zwei- oder dreifach substituiert sein, sofern dies aus sterischen Gründen oder aus Stabilitätsgründen möglich ist. Bei einer Nitrosubstitution ist in der Regel nur eine Nitrogruppe, gegebenenfalls neben anderen Substituenten, vorhanden.

Der Imidazolrest ist insbesondere ein 4-Imidazolrest. Der Indolylrest ist insbesondere ein 3-Indolylrest.

Geeignete substituierte Phenylreste sind z.B. 3,5-Di-bromo-4-hydroxy-phenyl.

$R^2$ und $R^3$ können auch mit dem Stickstoff, an das sie gebunden sind, einen 5- oder 6-gliedrigen gesättigten, heterocyclischen Ring bilden, der auch noch -O- oder $-N(R^5)-$ enthalten kann. Beispiele für derartige Reste sind 1-Pyrrolidinyl, Piperidino, Morpholino, 1-Piperazinyl, 4-($C_1$-$C_4$Alkyl)-piperazin-1-yl, insbesondere 4-Methyl-piperazin-1-yl, 4-Phenyl-piperazin-1-yl, 4-($C_1$-$C_4$-Alkoxy)-piperazin-1-yl.

Für $R^2$ ist Wasserstoff bevorzugt.

$R^4$ stellt den Rest einer natürlichen oder unnatürlichen Aminosäure oder eines Dipeptids dar, wobei dieser Rest formal durch Abspaltung einer $NH_2$-Gruppe aus der Aminosäure oder dem Dipeptid entstanden ist. Reste von β-Aminosäuren und insbesondere von α-Aminosäuren sind bevorzugt. Die Reste $R^4$ können beispielsweise von folgenden Aminosäuren abgeleitet sein, die falls chiral, in der D- oder L-Form vorliegen können (vgl. Houben-Weyl, Methoden der organischen Chemie, Band XV/1 und 2, Stuttgart, 1974):

Aad, Abu, γAbu, ABz, 2ABz, εAca, Ach, Acp, Adpd, Ahb, Aib, βAib, Ala, βAla, ΔAla, Alg, All, Ama, Amt, Ape,

Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)aminoessigsäure.

Der Rest $R^4$ kann auch von einem Dipeptid abgeleitet sein, wobei diese Dipeptide als Bausteine natürliche oder unnatürliche Aminosäuren enthalten können. Ferner können die Reste $R^4$ der natürlichen oder unnatürlichen Aminosäuren und Dipeptide auch als Ester bzw. Amide vorliegen, wie z. B. Methylester, Ethylamid, Semicarbazid oder ω-Amino-$(C_4$-$C_8)$-alkylamid.

Funktionelle Gruppen der Aminosäure- und Dipeptid-Reste $R^4$ können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z$(NO_2)$, Z$(Hal_n)$, Bobz, Iboc, Adpoc, Mboc, Acm, tert.-Butyl, OBzl, ONbzl, OMbzl, Bzl, Mob, Pic, Trt.

Für R ist -N$(R^2)R^3$ und insbesondere -NH-$R^4$ bevorzugt. Der Rest -N$(R^2)R^3$ bedeutet insbesondere einen Amino-$(C_1$-$C_8)$-alkyl-diphenylrest, einen Amino-$(C_3$-$C_8)$-cycloalkylrest oder einen Amino-$(C_1$-$C_8)$-alkylrest. Der Amino-$(C_3$-$C_8)$-cycloalkylrest kann dabei auch am Cycloalkylrest substituiert sein, insbesondere durch eine Carboxygruppe. Der Amino-$(C_1$-$C_8)$-alkyl-diphenylrest ist insbesondere ein ω,ω-Diphenylalkylaminrest, ganz besonders bevorzugt ein 3,3-Diphenylpropylaminrest. Besonders bevorzugt steht der für R stehende Rest -NH-$R^4$ für den Valin-, Phenylalanin- oder Phenylglyzinrest, der durch Abspaltung eines Wasserstoffatoms von der Aminogruppe des Valins, Phenylalanins oder Phenylglyzins entsteht.

Für $R^6$ sind Wasserstoff, Phenyl, Benzyl, Methyl oder Isopropyl bevorzugt.

Für k ist. 3 bevorzugt. Für p und q ist eine Zahl von 14 bis 16 bevorzugt.

Die für X stehenden Reste enthalten vorzugsweise 1,4-oder 1,3-Phenylen, p-Piperidinyl oder 3-Pyrrolidinyl.

Besonders bevorzugte Reste X sind:

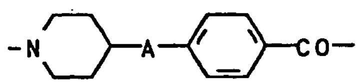
,

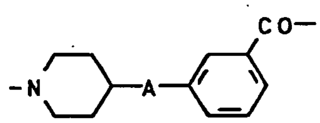
,

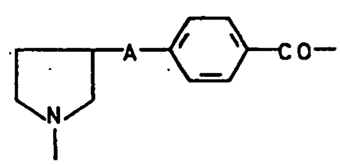
,

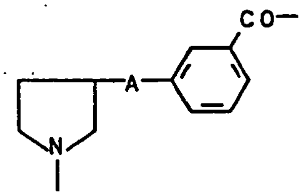
,

Bevorzugte Verbindungen der Formel I sind solche, die ein und insbesondere mehrere der bevorzugten Reste oder Gruppen enthalten.

Physiologisch verträgliche Salze der Verbindungen der allgemeinen Formel I sind insbesondere pharmazeutisch verwendbare oder nicht-toxische Salze.

Die Carboxylgruppe der Verbindungen der Formel I kann mit Alkali- oder Erdalkalimetallen, wie z.B. Na, K, Mg und Ca, sowie mit physiologisch verträglichen organischen Aminen, wie z.B. Triethylamin und Tris-(2-hydroxyethyl)-amin, Salze bilden.

Verbindungen der allgemeinen Formel I können auch an der basischen Amidinogruppe oder Guanidinogruppe mit anorganischen Säuren und mit organischen Carbon- oder Sulfonsäuren Salze bilden.

Verbindungen der Formel I können dadurch hergestellt werden, daß

a) eine Verbindung der allgemeinen Formel II

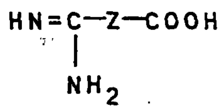

(II)

worin
Z

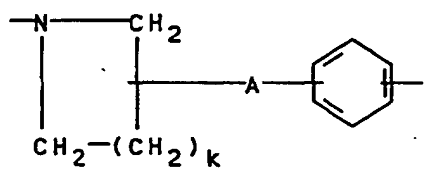

,

bedeutet und A die bereits angegebenen Bedeutungen besitzt , mit einer Verbindung der allgemeinen Formel III

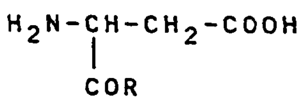

(III)

worin R die bereits genannte Bedeutung besitzt, umgesetzt wird, oder
b) eine Verbindung der allgemeinen Formel Ia

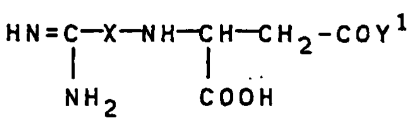

(Ia)

worin X die bereits eingangs genannte Bedeutung besitzt und $Y^1$ eine Schutzgruppe bedeutet, mit einer Verbindung der Formel IV

$$HR \qquad (IV)$$

worin R die bereits eingangs genannte Bedeutung besitzt, umgesetzt und dann die Schutzgruppe entfernt wird, und daß die Verbindung der Formel I gegebenenfalls in ein physiologisch verträgliches Salz übergeführt wird.

Die Umsetzungen zur Herstellung der erfindungsgemäßen Verbindungen der Formel I stellen im Prinzip

Acylierungsreaktionen dar. Sie werden nach bekannten Prinzipien, insbesondere nach den bekannten Methoden der Peptidchemie (vgl. z.B. Houben-Weyl, Methoden der organischen Chemie, Bd. 15/2 (1974); Ullmanns Enzyklopädie der techn. Chemie, 4. Auflage, Bd. 19, S.542-548) durchgeführt. Vorhandene Gruppen, die nicht reagieren sollen, werden durch Schutzgruppen geschützt, die nach der Umsetzung wieder abgespalten werden. Es können alle Schutzgruppen verwendet werden, die unter den Synthesebedingungen stabil sind und nach der Synthese der erfindungsgemäßen Verbindungen wieder abgespalten werden können. Geeignete Aminoschutzgruppen sind z.B. (in Klammer stehen die üblichen Abkürzungen): Benzyloxycarbonyl (Z), tert.Butyloxycarbonyl (Boc), 3,5-Dimethoxyphenylisopropyloxycarbonyl (Ddz), 2-(4-Biphenyl)-iso-propyloxycarbonyl (Bpoc), Trityl (Trt), Methylsulfonyl-ethyloxycarbonyl (Msc), 9-Fluorenylmethylcarbonyl (F-moc).

Guanidinogruppen können z.B. mit $NO_2$- oder mit Methoxytrimethyl-benzolsulfonyl (Mtr)-Gruppen geschützt werden. Als Carboxylschutzgruppen werden z.B. Methyl- oder Ethylester, tert.Butylester, Allyl-, Benzyl- oder Nitrobenzylester verwendet.

Als Schwefelschutzgruppen für im Molekül vorhandene Mercaptoreste sind z.B. Benzyl (Bzl), p-Methoxybenzyl (Mbzl), Trityl (Trt) oder Acetamidomethyl (Acm) geeignet.

Die Bedingungen für die Einführung und Abspaltung der Schutzgruppen sind aus der Peptidchemie bekannt (vgl. z.B. Houben-Weyl loc.cit., Ullmanns loc.cit.)

$NO_2$-Gruppen (Guanidinoschutz), Benzyloxycarbonylgruppen und Benzylestergruppen werden z.B. durch Hydrierung abgespalten. Schutzgruppen vom tert.Butyltyp werden durch saure Hydrolyse abgespalten.

Die Bedingungen für die Knüpfung der Peptidbindung sind ebenfalls aus der Peptidchemie bekannt (vgl. z. B. Houben-Weyl loc.cit., Ullmanns loc.cit.).

Bei der Bildung der Amid- bzw. Peptidbindung ist eine direkte Wasserabspaltung bei der Reaktion der Aminokomponente mit der -COOH-haltigen Komponente durch Zugabe geeigneter Kondensationsmittel möglich, wobei in wasserfreiem Medium gearbeitet wird. Zweckmäßigerweise wird jedoch vor oder während der Umsetzung eine Aktivierung der Carboxylgruppe durchgeführt. Die Aktivierung der Carboxylgruppe kann z.B. nach der Azidmethode oder der Methode der gemischten Anhydride erfolgen. Bei der Methode der gemischten Anhydride werden z.B. Halbester der Kohlensäure verwendet. Besonders bequem ist die Aktivierung der Carboxylgruppe nach der Carbodiimidmethode, z.B. unter Verwendung von Dicyclohexylcarbodiimid (DCC), wobei die Reaktion im Eintopfverfahren durchgeführt werden kann. Zweckmäßigerweise wird die Carbodiimidmethode mit der Aktivestermethode kombiniert, d.h. zusätzlich zu einem Carbodiimid wird eine einen Aktivester bildende N-Hydroxyverbindung, wie z.B. N-Hydroxy-succinimid (HONSu), 1-Hydroxy-benzotriazol (HOBt) oder 3-Hydroxy-4-oxo-3,4-dihydro-1,2,3-benzotriazin (HOObt), eingesetzt. Die Verwendung von HOBt oder HOObt in Kombination mit einem Carbodiimid, insbesondere DCC, ist bevorzugt.

Die Umsetzungen werden zweckmäßigerweise in einem geeigneten inerten Lösungs- oder Dispergiermittel, wie z.B. Wasser, Methanol, Ethanol, Acetonitril, einem Amid, wie Dimethylformamid, Dimethylacetamid, N-Methylpyrrolidon, Methylenchlorid oder einem Ether, wie Tetrahydrofuran, oder einem Gemisch verschiedener Lösungs- oder Dispergiermittel durchgeführt.

Die Umsetzungen können prinzipiell bei Temperaturen zwischen -10°C und dem Siedepunkt des verwendeten Lösungs- oder Dispergiermittels erfolgen. In vielen Fällen wird die Umsetzung bei 0 bis 50°C, insbesondere bei 0 bis 30°C und vorzugsweise bei 0 bis Raumtemperatur, durchgeführt.

Bei der Herstellung der Verbindungen der Formel I werden die Ausgangskomponenten sowie das gegebenenfalls zur Anwendung kommende Aktivierungsmittel oder Aktivierungsmittelgemisch normalerweise in etwa äquimolaren Mengen eingesetzt.

Die erfindungsgemäßen Verbindungen der Formel I bilden mit anorganischen oder organischen Säuren Säureadditionssalze. Zur Bildung derartiger Säureadditionssalze geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Glykol-, Sorbin-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen-oder Adipin-Säure. Die Säureadditionssalze werden wie üblich durch Vereinigung der Komponenten, zweckmäßigerweise in einem geeigneten Lösungsmittel oder Verdünnungsmittel, hergestellt.

Carboxylgruppen in den Verbindungen der Formel I können durch Vereinigen in bekannter Weise mit Hydroxiden, wie z.B. Natrium-, Kalium-, Rubidium-, Lithium-, Magnesium-, Calciumhydroxid oder organischen Aminen, Salze bilden.

Die Ausgangsverbindungen der Formel II können aus den entsprechenden Aminoverbindungen durch Guanylierung oder Nitroguanylierung hergestellt werden. Die Aminoverbindungen sind bekannt bzw. können aus den entsprechenden bekannten Ausgangsverbindungen nach üblichen Methoden hergestellt werden. Für die Guanylierung und Nitroguanylierung können folgende Reagentien verwendet werden:

1. O-Methylisoharnstoff (S.Weiss und H.Krommer, Chemiker Zeitung 98 (1974) 617-618);

2. S-Methylisothioharnstoff (R.F.Borne, M.L.Forrester und I.W.Waters, J.Med.Chem. 20 (1977) 771-776);
3. Nitro-S-methylisothioharnstoff (L.S.Hafner und R.E.Evans, J.Org.Chem. 24 (1959) 1157);
4. Formamidinsulfonsäure (K.Kim, Y.-T.Lin und H.S.Mosher, Tetrahedron Lett. 29 (1988) 3183-32186);
5. 3,5-Dimethyl-1-pyrazolyl-formamidinium-nitrat (F.L.Scott, D.G. O'Donovan und J.Reilly, J.Amer.Chem.Soc. 75 (1953) 4053-4054).

Die Ausgangsverbindungen der Formeln III werden in bekannter Weise in der Regel vom C-terminalen Ende her stufenweise aus bekannten Verbindungen aufgebaut. Die Peptidknüpfungen können mit den bekannten Kupplungsmethoden der Peptidchemie durchgeführt werden.

Die Verbindungen der Formel III können z.B. in an sich bekannter Weise hergestellt werden durch Umsetzung einer Verbindung der allgemeinen Formel IIIb

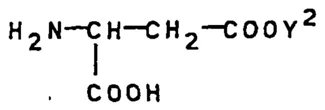

(IIIb)

mit einer Verbindung HR.

Ausgangsverbindungen der Formel Ia werden analog den erfindungsgemäßen Verbindungen aufgebaut. Die Ausgangsverbindungen der Formel IV sind bekannt oder können nach bekannten Methoden hergestellt werden.

Die erfindungsgemäßen neuen Asparaginsäure-Derivate der Formel I und ihre physiologisch verträglichen Salze haben die Fähigkeit, die Bindung von Fibrinogen, Fibronectin und des von Willebrand Faktors an Integrinrezeptoren zu hemmen.

Integrine sind Zellmembran-Glykoproteine und vermitteln Zelladhäsion durch Wechselwirkung mit einer Vielzahl extrazellulärer Proteine, wie Fibronectin, Laminin, Fibrinogen, Kollagen, Vitronectin, und von Willebrand Faktor oder mit anderen Zellmembranproteinen, wie z.B. ICAM-1. Ein wichtiger Rezeptor aus der Integrinfamilie ist das auf Blutplättchen lokalisierte Glykoprotein IIb/IIIa (Fibrinogen-Rezeptor) - ein Schlüsselprotein der Plättchen-Plättchen-Wechselwirkung und Thrombenbildung. Ein zentrales Fragment in der Rezeptorerkennungssequenz dieser Proteine ist das Tripeptid Arg-Gly-Asp (E.Ruoslahti und M.D.Pierschbacher, Science 238 (1987) 491-497; D.R.Phillips, I.F.Charo, L.V.Parise und L.A.Fitzgerald, Blood 71 (1988) 831-843).

Die erfindungsgemäßen Verbindungen der allgemeinen Formel I und ihre physiologisch verträglichen Salze hemmen die Thrombozytenaggregation, die Metastasierung von Karzinomzellen sowie die Osteoclastenbindung an die Knochenoberflächen.

Die Verbindungen der Formel I und ihre physiologisch annehmbaren Salze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes davon, neben einem oder mehreren üblichen pharmazeutisch einwandfreien Träger-, Füll- oder Verdünnungs- und gegebenenfalls einem oder mehreren Zusatzstoffen enthalten.

Die Heilmittel können oral, z.B. in Form von Tabletten, Filmtabletten, Dragees, Hart- und Weichgelatinekapseln, Mikrokapseln, Granulaten, Pulvern, Pellets, Lösungen, Sirupen, Emulsionen, Suspensionen, Aerosolen, Schäumen, Pillen oder Pastillen verabreicht werden. Die Verabreichung kann aber auch rektal, z.B. in Form von Suppositorien, oder parenteral, z.B. in Form von Injektionslösungen, oder perkutan, z.B. in Form von Salben, Cremes, Gelen, Pasten, Aerosolen, Schäumen, Pudern, Tinkturen, Linimenten oder sogenannten transdermalen therapeutischen Systemen (TTS) oder nasal, z.B. in Form von Nasalsprays, erfolgen.

Die pharmazeutischen Präparate können in an sich bekannter Weise unter Verwendung pharmazeutisch inerter anorganischer oder organischer Hilfs-, Träger-, Füll- oder Verdünnungsstoffe hergestellt werden. Für die Herstellung von Pillen, Tabletten, Filmtabletten, Dragees und die Pellet- oder Granulatfüllungen von Hartgelatinekapseln kann man z.B. Calciumphosphate, Lactose, Sorbitol, Mannitol, Stärken, präparierte Stärken, chemisch modifizierte Stärken, Stärkehydrolysate, Cellulose, Cellulosederivate, synthetische Polymere, Talk etc. verwenden. Träger- oder Verdünnungsstoffe für Weichgelatinekapseln und Suppositorien sind z.B. Fette, Wachse, halbfeste und flüssige Polyole, natürliche oder gehärtete Öle etc. Als Träger- oder Verdünnungsstoffe für die Herstellung von Lösungen und Sirupen eignen sich z.B. Wasser, Polyole, Lösungen von Saccharose, Invertzucker, Glukose etc. Als Trägerstoffe für die Herstellung von Injektionslösungen eignen sich z.B. Wasser, Alkohole, Glyzerol, Polyole oder pflanzliche Öle. Als Träger- oder Verdünnungsstoffe für Salben, Cremes und Pasten eignen sich z.B. Naturvaseline, Kunstvaseline, dick- und dünnflüssige Paraffine, Fette, natürliche oder gehärtete pflanzliche und tierische Öle, Neutralöle, Wachse, Wachsalkohole, Polyethylenglykole, Polyacrylsäure, Silicongele etc. Als Trägerstoffe für Mikrokapseln oder Implantate eignen

sich z.B. Mischpolymerisate aus Glykolsäure und Milchsäure.

Die pharmazeutischen Präparate können in an sich bekannter Weise neben den Wirk- und Verdünnungs- oder Trägerstoffen auch noch einen oder mehrere Zusatzstoffe oder Hilfsmittel, wie z.B. Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler, Lösungsbeschleuniger, Antischaummittel, Salzbildner, Gelbildner, Verdickungsmittel, Fließregulierungsmittel, Sorptionsmittel, Mittel zur Erzielung eines Depoteffekts oder Mittel, insbesondere Salze, zur Veränderung des osmotischen Drucks, Überzugsmittel oder Antioxidantien etc. enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanthano-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metaprolol und Penbutolol. Darüberhinaus lassen sich die Verbindungen mit anderen nootrop wirksamen Substanzen, wie z.B. Piracetam, oder ZNS-aktiven Substanzen, wie Pirlindol, Sulpirid etc. kombinieren.

In den pharmazeutischen Präparaten kann der Gehalt an dem Wirkstoff oder den Wirkstoffen der Formel I in weiten Grenzen schwanken und z.B. 0,05 bis 50 Gew.%, vorzugsweise 0,05 bis 20 Gew.%, betragen. In festen Darreichungsformen, wie Dragees, Tabletten etc. beträgt der Gehalt an einem oder mehreren Wirkstoffen der Formel I in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis 1 Gew.%, eines oder mehrerer Wirkstoffe der Formel I. Der Gehalt an einem oder mehreren Wirkstoffen der Formel I kann gegebenenfalls in den pharmazeutischen Präparaten teilweise, z.B. bis zu 50 Gew.%, vorzugsweise zu 5 bis 40 Gew.%, durch eine oder mehrere andere therapeutisch wirksame Substanzen ersetzt sein.

Die Verbindungen der Formel I, ihre physiologisch annehmbaren Salze und die pharmazeutischen Präparate, welche die Verbindungen der Formel I oder ihre physiologisch annehmbaren Salze als Wirkstoffe enthalten, können am Menschen zur Prophylaxe und Therapie von z.B. arteriellen Gefäßerkrankungen, wie akutem Myokardinfarkt in Kombination mit Lysetherapie, post-Infarktbehandlung, Sekundärprävention des Myokardinfarktes, Reocclusionsprophylaxe nach Lyse und Dilatation, instabiler Angina Pectoris, transitorischer ischämischer Attacken, Schlaganfall, koronarer Bypass-Operation und Reocclusionsprophylaxe des Bypasses, Lungenembolie, peripherer arterieller Verschlußkrankheiten, dissezierendem Aneurysma, zur Therapie venöser und mikrozirkulatorischer Gefäßerkrankungen, wie tiefer Venenthrombose, disseminierter intravaskulärer Gerinnung, post-operativem und post-partum Trauma, chirurgischem oder infektiösem Schock, Septicämie, zur Therapie bei Erkrankungen mit hyperreagiblen Thrombozyten, thrombotische thrombozytopenische Purpura, Preeklampsie, prämenstruelles Syndrom, Dialyse, extrakorporale Zirkulation, ferner bei Entzündungen und bei der Behandlung von Tumoren und der Hemmung der Osteoclastenbindung an die Knochenoberfläche eingesetzt werden.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4 Teilverabreichungen aufgeteilt.

Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer pharmazeutisch akzeptablen Säureadditionssalze pro Dosis.

Geprüft werden die Verbindungen der Formel I vor allem auf ihre hemmende Wirkung bei der Blutplättchenaggregation und der Anhaftung von Fibrinogen an Blutplättchen. Die Messung der Aggregation und der Bindung von $^{125}$I-Fibrinogen wird an gefiltierten, plasmafreien Human-Thrombozyten durchgeführt. Die Aktivierung der Thrombozyten erfolgt durch ADP oder Thrombin.

Als funktioneller Test wird die Hemmung der Aggregation gefiltrierter Human-Thrombozyten nach ADP- oder Thrombin-Stimulierung durch die erfindungsgemäßen Verbindungen gemessen. Angegeben ist der $IC_{50}$-Wert der Hemmung.

Literatur: G.A. Marguerie et al., J. Biol. Chem. 254 (1979), 5357-5363 Angegeben ist der $K_i$-Wert der Bindungshemmung von $^{125}$I-Fibrinogen nach Stimulierung mit ADP (10 μM).

Literatur: J.S. Bennett u. G. Vilaire, J. Clin. Invest. 64, (1979), 1393-1401 E. Kornecki et al., J. Biol. Chem. 256 (1981), 5695-5701 G.A. Marguerie et al., J. Biol. Chem. 254 (1979), 5357-5363 G.A. Marguerie et al., J. Biol.

Chem. 255 (1980), 154-161

Bei der Prüfung der Hemmung der Fibrinogenbindung und der Hemmung der Aggregation werden für die Verbindungen der nachfolgenden Beispiele folgende Ergebnisse erhalten:

| Beispiel | Plättchenaggregationshemmung | | Hemmung der Fibrinogenbindung |
|---|---|---|---|
| | ADP (μM) | Thrombin (μM) | $K_i$ (μM) |
| 1 | 8 | 2 | * |
| 2 | 15 | 10 | * |
| 3 | 15 | 5 | * |
| 4 | 20 | 15 | 10 |
| 14 | 4.5 | 1.5 | 0.8 |

*: nicht gemessen

In den nachstehenden Beispielen werden u.a. folgende Abkürzungen verwendet:

Asp = Asparaginsäure
Val = Valin
Bn = Benzyl
HOBt = 1-Hydroxy-benzotriazol
DMF = Dimethylformamid
DCC = Dicyclohexyl-carbodiimid
DCH = Dicyclohexylharnstoff

**BEISPIELE**

Beispiel 1:

**p-(4-(N-Amidinopiperidinyl)-methyl)-benzoyl-L-aspartyl-L-valin**

a) p-(4-(Nitroamidinopiperidinyl)-methyl)-benzoesäure

Zur Lösung von 4,96 g (22,6 mmol) p-(4-Piperidinylmethyl)-benzoesäure und 1,81 g (45,2 mmol) NaOH in 50 ml Wasser werden bei 0°C unter Rühren 3,42 g (25,3 mmol) Nitro-S-methylisothioharnstoff zugesetzt. Nach 24 h Rühren bei Raumtemperatur wird mit konz. HCl auf pH = 1 angesäuert. Der Niederschlag wird abfiltriert, mit Wasser gewaschen und getrocknet.
Ausbeute: 6,13 g (89 %);
Schmelzpunkt: 260 bis 261°C (Zers.).

b) p-(4-(N-Nitroamidinopiperidinyl)-methyl)-benzoyl-Asp(OBn)-Val-OBn

Zur Lösung von 1,11 g (3,64 mmol) p-(4-(Nitroamidinopiperidinyl)-methyl)-benzoesäure, 1,50 g (3,64 mmol) $H_2N$-Asp(OBn)-Val-OBn und 0,49 g (3,64 mmol) HOBt in 25 ml DMF gibt man bei 0°C 0,83 g (3,64 mmol) DCC. Nach 4 h Rühren bei 0°C wird weitere 12 h bei Raumtemperatur gerührt. Es wird vom ausgefallenen DCH abfiltriert und der Rückstand chromatographisch gereinigt.
Ausbeute: 2,43 g (95 %), enthält noch etwas DCH;
Schmelzpunkt: 60 bis 75°C.

c) p-(4-(N-Amidinopiperidinyl)-methyl)-benzoyl-L-aspartyl-L-valin

2,29 g (3,27 mmol) p-(4-(N-Nitroamidinopiperidinyl) methyl)-benzoyl-Asp(OBn)-Val-OBn werden in 20 ml Methanol/10 ml DMF gelöst und mit 0,25 g Pd/C (10 %) versetzt. Nach 6 h Hydrieren bei Raumtemperatur werden 20 ml Wasser und 20 ml Eisessig zugesetzt und weitere 1,5 h hydriert. Vom Katalysator wird abfiltriert, der Rückstand über ®Sephadex LH-20 chromatographiert (Methanol).

Ausbeute: 1,47 g (94 %);
Schmelzpunkt: 260 bis 270°C.
$(\alpha)_D^{20}$ = -5,5° (c = 1 in Eisessig)

Beispiel 2:

**p-(4-(N-Amidinopiperidinyl)-methyl)-benzoyl-L-aspartylcyclohexylamid**

a) p-(4-(N-Nitroamidinopiperidinyl)-methyl)-benzoyl-Asp(OBn)-cyclohexylamid

Analog 1b) läßt man 1,51 g (4,93 mmol) p-(4-(Nitroamidinopiperidinyl)-methyl)-benzoesäure mit 1,50 g (4,93 mmol) $H_2N$-Asp(OBn)-cyclohexylamid reagieren.
Ausbeute: 2,3 g (79 %);
Schmelzpunkt: 70 bis 75°C.

b) p-(4-N-Amidinopiperidinyl)-methyl)-benzoyl-L-aspartyl-cyclohexylamid

2,1 g (3,54 mmol) p-(4-N-Nitroamidinopiperidinyl)-methyl)-benzoyl-Asp(OBn)-cyclohexylamid werden analog 1c) hydriert.
Ausbeute: 1,6 g (82 %);
Schmelzpunkt: 220 bis 225°C;
$(\alpha)_D^{20}$ = -5,8° (c = 1,2 in Eisessig)

Beispiel 3:

**p-(4-N-Amidinopiperidinyl)-methyl)-benzoyl-L-aspartyl-3,3-diphenylpropylamid**

a) p-(4-N-Nitroamidinopiperidinyl)-methyl)-benzoyl-Asp(OBn)-3,3-diphenylpropylamid

Analog 1b) läßt man 1,1 g (3,6 mmol) p-(4-(Nitroamidinopiperidinyl)-methyl)-benzoesäure mit 1,50 g (3,6 mmol) $H_2N$-Asp(OBn)-3,3-diphenylpropylamid reagieren.
Ausbeute: 2,15 g (85 %) Acetat;
Schmelzpunkt: 90 bis 100°C.

b) p-(4-N-Amidinopiperidinyl)-methyl)-benzoyl-L-aspartyl-3,3-diphenylpropylamid

1,97 g (2,8 mmol) p-(4-(N-Nitroamidinopiperidinyl)-methy)l-benzoyl-Asp(OBn)-3,3-diphenylpropylamid werden analog 1c) hydriert.
Ausbeute: 1,57 g (89 %) Acetat;
Schmelzpunkt: 160 bis 175°C;
$(\alpha)_D^{20}$ = -6,4° (c = 1,1 in Eisessig)
Analog wurden hergestellt:

Beispiel 4:

**p-(4-N-Amidinopiperidinyl)-methyl)-benzoyl-L-aspartylisopropylamid**

Schmelzpunkt: 206 bis 208°C
$(\alpha)_D^{20}$ = +5,6° (c = 1,2 in Eisessig)

Beispiel 14:

**p-(4-N-Amidinopiperidinyloxy)-benzoyl-L-aspartyl-L-valin**

a) p(4-N-Nitroamidinopiperidinyloxy)-benzoyl-L-Asp(OBn)-L-Val-OBn

Analog 1b) läßt man 0,3 g (= 0,97 mmol) p-(4-N-Nitroamidinopiperidinyloxy)-benzoesäure mit 0,4 g (0,97 mmol) $H_2N$-Asp(OBn)-Val-OBn reagieren.
Ausbeute: 0,55 g (81 %).

b) p-(4-N-Amidinopiperidinyloxy-benzoyl-L-aspartyl-L-valin

0,36 g (0,51 mmol) p-(4-N-Nitroamidinopiperidinyloxy)-benzoyl-L-Asp(OBn)-L-Val-OBn werden analog 1c) hydriert. Ausbeute: 0,23 g (94 %),
Schmelzpunkt: 150°C.

Beispiel 15:

**p-4-N-Amidinopiperidinyl-benzoyl-L-aspartyl-L-valin**

a) p-4-Nitroamidinopiperidinyl-benzoyl-Asp(OBn)-L-Val-OBn

Analog 1b) läßt man 2,5 g (8,55 mmol) p-4-Nitroamidinopiperidinyl-benzoesäure mit 3,56 g (8,55 mmol) $H_2N$-Asp (OBn)-Val-OBn reagieren.
Ausbeute: 1,92 g (25 %).

b) p-4-N-Amidinopiperidinyl-benzoyl-L-aspartyl-L-valin

Analog 1c) werden 0,42 g (0,61 mmol) p-4-Nitroamidinopiperidinyl-benzoyl-Asp(OBn)-L-Val-OBn hydriert.
Ausbeute: 0,14 g (41 %);
Schmelzpunkt: 190°C (Zers.)

Beispiel 16:

**p-(3-N-Amidinopyrolidinyloxy)-benzoyl-L-aspartyl-L-valin**

a) p-(3-N-Nitroamidinopyrolidinyloxy)-benzoyl-L-Asp(OBn) -L-Val-OBn

Analog 1b) läßt man 0,29 g (0,99 mmol) p-(3-N-Nitroamidinopyrolidinyloxy)-benzoesäure mit 0,41 g (0,99 mmol) $H_2N$-Asp(OBn)-Val-OBn reagieren.
Ausbeute: 0,63 g (92 %).

b) p-(3-N-Amidinopyrolidinyloxy)-benzoyl-L-aspartyl-L-valin

0,63 g (0,92 mmol) p-(3-N-Nitroamidinopyrolidinyloxy)-benzoyl-L-Asp(OBn)-L-Val-OBn werden analog 1c) hydriert. Ausbeute: 0,11 g (27 %);
Schmelzpunkt: 250°C;
$(\alpha)_D^{20}$ = -5,95° (C = 0,84 in Methanol/Wasser 2:1).

Die nachfolgenden Beispiele A bis H betreffen pharmazeutische Zubereitungen.

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |

(fortgesetzt)

| | |
|---|---|
| Polyoxyethylenstearat | q. s. |
| Reinglycerin | 0,6 bis 2 g |
| Aromastoffe | q. s. |
| Wasser (entmineralisiert oder destillierts) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

**Patentansprüche**

**1.** Asparaginsäurederivate der allgemeinen Formel I

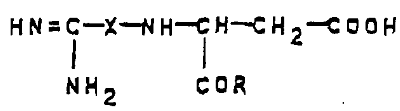

(I)

worin

X

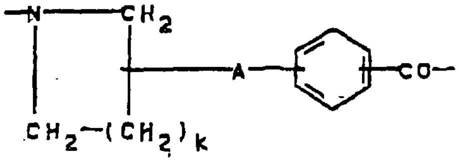

,

A -$(CH_2)_m$-, -O- oder eine direkte Bindung,
k die Zahl 2 oder die Zahl 3,
m die Zahl 1,

R -$OR^1$,

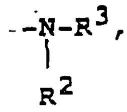

-NH-$R^4$,

$R^1$ Wasserstoff, $C_1$-$C_{28}$Alkyl, $C_3$-$C_{28}$Cycloalkyl, Phenyl wobei das $C_1$-$C_{28}$Alkyl, das $C_3$-$C_{28}$Cycloalkyl oder das Phenyl unsubstituiert oder ein- oder mehrfach mit gleichen oder verschiedenen Resten aus der Reihe Hydroxy, Carboxy, $C_1$-$C_4$Alkoxycarbonyl, Arylmethoxycarbonyl, Carboxamido, $C_1$-$C_4$Alkylaminocarbonyl, Amino, Mercapto, $C_1$-$C_4$-Alkoxy, $C_3$-$C_8$Cycloalkyl, Imidazolyl, Indolyl, Pyrrolidinyl, Hydroxypyrrolidinyl, Halogen, unsubstituiertem oder ein oder mehrfach mit Hydroxy, $C_1$-$C_4$Alkyl, Halogen, Nitro oder Trifluormethyl, substituiertem Phenyl oder Phenoxy, substituiert ist, ein- oder mehrfach durch -O-unterbrochenes $C_3$-$C_{81}$Alkyl oder ein- oder mehrfach durch -O- unterbrochenes ($C_3$-$C_{81}$Alkoxy)carbonyl; $R^2$, $R^3$ unabhängig voneinander Wasserstoff, $R^1$ oder zusammen mit dem Stickstoff, an dem sie gebunden sind, einen 5- oder 6-gliedrigen, gesättigten heterocyclischen Ring, der auch noch -O- oder -$NR^5$- enthalten kann, oder $R^2$ Wasserstoff und $R^3$ einen Rest der Formel

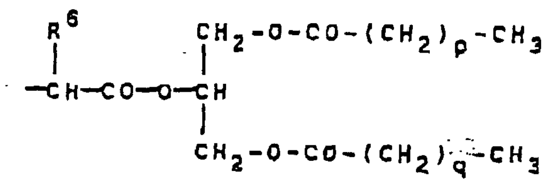

oder

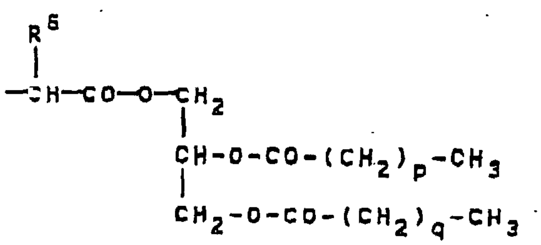

$R^4$ den Rest einer Aminosäure oder eines Dipeptids, bei dem die Peptidbindung auch zu -NH-$CH_2$- reduziert sein kann,
$R^5$ Wasserstoff, $C_1$-$C_4$Alkyl, Phenyl, $C_1$-$C_4$Alkylphenyl, $C_1$-$C_4$Alkoxyphenyl,
$R^6$ Wasserstoff, $C_1$-$C_4$Alkyl, Phenyl, Phenyl($C_1$-$C_4$)Alkyl,
p, q unabhängig voneinander eine Zahl von 1 bis 20 bedeuten, sowie deren physiologisch verträglichen Salze.

**2.** Verbindungen nach Anspruch 1, **dadurch gekennzeichnet, daß** die für X stehenden Reste 1,4- oder 1,3-Phenylen enthalten.

**3.** Verbindungen nach Anspruch 1 und/oder 2, **dadurch gekennzeichnet, daß** die für X stehenden Reste p-Piperidinyl oder 3-Pyrrolidinyl enthalten.

**4.** Verbindungen nach einem oder mehreren der Ansprüche 1 bis 3, **dadurch gekennzeichnet, daß** R -N($R^2$)$R^3$ oder -$NHR^4$ bedeutet.

**5.** Verbindungen nach einem oder mehreren der Ansprüche 1 bis 4, **dadurch gekennzeichnet, daß** R -$NHR^4$ be-

deutet, wobei $R^4$ für den Valin-, Phenylalanin- oder Phenylglycinrest steht, der formal durch Abspaltung der Aminogruppe aus dem Valin, Phenylalanin oder Phenylglycin entsteht.

**6.** Verfahren zur Herstellung von Asparaginsäurederivaten eines oder mehrerer der Ansprüche 1 bis 5, **dadurch gekennzeichnet, daß**

a) eine Verbindung der allgemeinen Formel II

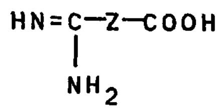 (II)

worin Z

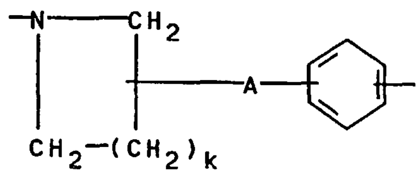 ,

bedeutet und A und k die in den Ansprüchen 1 bis 5 angegebenen Bedeutungen besitzen, mit einer Verbindung der allgemeinen Formel III

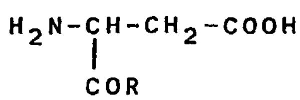 (III)

worin R die in den Ansprüchen 1 bis 5 genannte Bedeutung besitzt, umgesetzt wird, oder
b) eine Verbindung der allgemeinen Formel Ia

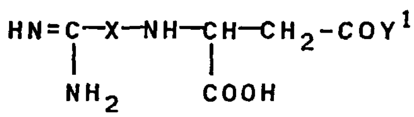 (Ia)

worin X die bereits eingangs genannte Bedeutung besitzt und $Y^1$ eine Schutzgruppe bedeutet, mit einer Verbindung de Formel IV

HR (IV)

worin R die bereits eingangs genannte Bedeutung besitzt, umgesetzt und dann die Schutzgruppe entfernt wird, und daß die Verbindung der Formel I gegebenenfalls in ein physiologisch verträgliches Salz übergeführt wird.

**7.** Verfahren nach Anspruch 6, **dadurch gekennzeichnet, daß** Aminogruppen, Amidinogruppen, Guanylgruppen, Merkaptogruppen und/oder Carboxylgruppen in den eingesetzten Ausgangsprodukten Schutzgruppen tragen, die gegebenenfalls nach beendeter Umsetzung in an sich bekannter Weise abgespalten werden.

**8.** Verbindung eines oder mehrerer der Ansprüche 1 bis 5 als Hemmstoff der Thrombozytenaggregation, der Metastasierung von Karzinomzellen oder der Osteoclastenbindung an die Knochenoberfläche.

**9.** Pharmazeutisches Präparat, **dadurch gekennzeichnet, daß** es eine oder mehrere Verbindungen der allgemeinen Formel I eines oder mehrerer der Ansprüche 1 bis 5 oder ein physiologisch verträgliches Salz davon als Wirkstoff zusammen mit pharmazeutisch annehmbaren Träger- und Zusatzstoffen und gegebenenfalls noch ein oder mehrere andere pharmakologische Wirkstoffe enthält.

**10.** Verfahren zur Herstellung eines pharmazeutischen Präparates, enthaltend eine oder mehrere Verbindungen der allgemeinen Formel I eines oder mehrerer der Ansprüche 1 bis 5 oder ein physiologisch verträgliches Salz davon, **dadurch gekennzeichnet, daß** man diese zusammen mit pharmazeutisch annehmbaren Träger- und Zusatzstoffen und gegebenenfalls noch ein oder mehrere andere pharmakologische Wirkstoffe in eine geeignete Darreichungsform bringt.

## Revendications

**1.** Dérivés de l'acide aspartique de formule générale I

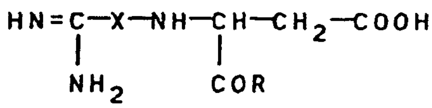

(I)

dans laquelle

X signifie

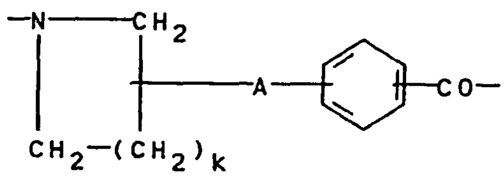

,

A signifie $-(CH_2)_m-$, -O- ou une liaison directe,
k signifie le nombre 2 ou le nombre 3,
m signifie le nombre 1
R signifie $-OR^1$,

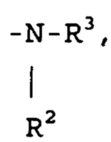

$-NH-R^4$,

$R^1$ signifie hydrogène, alkyle en $C_1$ à $C_{28}$, cycloalkyle en $C_3$ à $C_{28}$, phényle, où alkyle en $C_1$ à $C_{28}$, cycloalkyle en $C_3$ à $C_{28}$ ou phényle sont non substitués ou monosubstitués ou polysubstitués par radicaux identiques ou différents de la série hydroxy, carboxy, (alcoxy en $C_1$ à $C_4$)carbonyle, arylméthoxycarbonyle, carboxamido, (alkyle en $C_1$ à $C_4$)aminocarbonyle, amino, mercapto, alcoxy en $C_1$ à $C_4$, cycloalkyle en $C_3$ à $C_8$, imidazolyle, indolyle, pyrrolidinyle, hydroxypyrrolidinyle, halogène, phényle ou phénoxy non substitué ou monosubstitué ou polysubstitué par hydroxy, alkyle en $C_1$ à $C_4$, halogène, nitro ou trifluorométhyle ; alkyle en $C_3$ à $C_{81}$ interrompu une ou plusieurs fois par -O- ; ou (alcoxy en $C_3$ à $C_{81}$)carbonyle interrompu une ou plusieurs fois par -O-,
$R^2$ et $R^3$ signifient, indépendamment l'un de l'autre, hydrogène, $R^1$ ou, avec l'atome d'azote auquel ils sont liés, un hétérocycle saturé, de 5 à 6 chaînons, qui peut également encore contenir -O- ou $-NR^5-$,

ou $R^2$ signifie hydrogène et $R^3$ un radical de formule

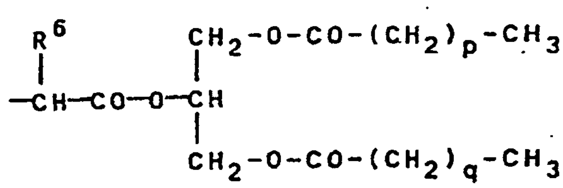

ou

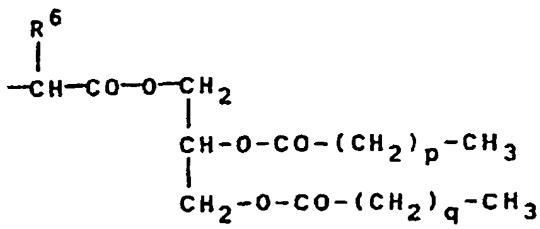

$R^4$ signifie le résidu d'un acide aminé ou d'un dipeptide, dans lequel la liaison peptidique peut également être réduite à $-NH-CH_2-$,
$R^5$ signifie hydrogène, alkyle en $C_1$ à $C_4$, phényle, (alkyle en $C_1$ à $C_4$)phényle, (alcoxy en $C_1$ à $C_4$)phényle,
$R^6$ représente hydrogène, alkyle en $C_1$ à $C_4$, phényle, phényl(alkyle en $C_1$ à $C_4$),
p et q signifient, indépendamment l'un de l'autre, un nombre de 1 à 20, ainsi que leurs sels physiologiquement acceptables.

**2.** Composés selon la revendication 1, **caractérisés en ce que** les radicaux représentés par X contiennent 1,4-phénylène ou 1,3-phénylène.

**3.** Composés selon la revendication 1 et/ou 2, **caractérisés en ce que** les radicaux représentés par X contiennent p-pipéridinyle ou 3-pyrrolidinyle.

**4.** Composés selon l'une ou plusieurs des revendications 1 à 3, **caractérisés en ce que** R signifie $-N(R^2)R^3$ ou $-NHR^4$.

**5.** Composés selon l'une ou plusieurs des revendications 1 à 4, **caractérisés en ce que** R signifie $-NHR^4$, où $R^4$ représente le résidu valine, phénylalanine ou phénylglycine, qui est formé formellement par dissociation du groupe amino de la valine, la phénylalanine ou la phénylglycine.

**6.** Procédé pour la préparation de dérivés d'acide aspartique selon l'une ou plusieurs des revendications 1 à 5, **caractérisé**

a) en ce qu'on transforme un composé de formule générale II

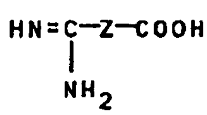

(II)

dans laquelle Z signifie

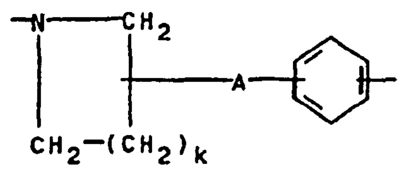 ,

et A et k ont les significations indiquées dans les revendications 1 à 5, avec un composé de formule générale III

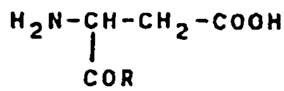 (III)

dans laquelle R présente les significations mentionnées dans les revendications 1 à 5, ou b) en ce qu'on transforme un composé de formule générale Ia

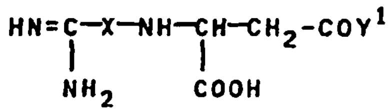 . (Ia)

dans laquelle X présente la signification déjà mentionnée dans l'introduction et $Y^1$ signifie un groupe de protection, avec un composé de formule IV dans laquelle R présente la signification déjà mentionnée dans l'introduction, puis le groupe de protection est éliminé et en ce que le composé de formule I est le cas échéant transformé en un sel physiologiquement acceptable.

**7.** Procédé selon la revendication 6, **caractérisé en ce que** les groupes amino, amidino, guanyle, mercapto et/ou carboxyle dans les produits de départ utilisés portent des groupes de protection qui sont le cas échéant dissociés de manière connue en soi à la fin de la transformation.

**8.** Composé selon l'une ou plusieurs des revendications 1 à 5 comme inhibiteur de l'agrégation des thrombocytes, de la formation de métastases de cellules carcinomateuses ou de la liaison d'ostéoclastes à la surface des os.

**9.** Préparation pharmaceutique, **caractérisée en ce qu'**elle contient un ou plusieurs composés de formule générale I selon l'une ou plusieurs des revendications 1 à 5 ou un sel physiologiquement acceptable de ceux-ci comme substance active, avec des substances support et des additifs pharmaceutiquement acceptables et le cas échéant encore une ou plusieurs autres substances actives pharmacologiques.

**10.** Procédé pour la préparation d'une préparation pharmaceutique, contenant un ou plusieurs composés de formule générale I selon l'une ou plusieurs des revendications 1 à 5 ou un sel physiologiquement acceptable de ceux-ci, **caractérisé en ce qu'**on les amène, avec des substances support et des additifs pharmaceutiquement acceptables et le cas échéant encore une ou plusieurs autres substances actives pharmacologiques, dans une forme d'administration appropriée.

## Claims

**1.** Aspartic acid derivatives of the general formula I

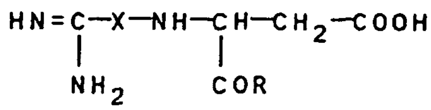 (I)

in which

x denotes

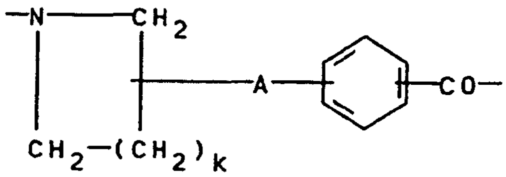 ,

A denotes $-(CH_2)_m-$, -O- or a direct bond,
k denotes the number 2 or the number 3,
m denotes the number 1,
R denotes $-OR^1$,

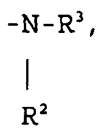

$-NH-R^4$,

$R^1$ denotes hydrogen, $C_1$-$C_{28}$alkyl, $C_3$-$C_{28}$cycloalkyl, phenyl, where the $C_1$-$C_{28}$alkyl, the $C_3$-$C_{28}$cycloalkyl or the phenyl is unsubstituted or mono- or polysubstituted by identical or different radicals from the series consisting of hydroxyl, carboxyl, $C_1$-$C_4$alkoxycarbonyl, arylmethoxycarbonyl, carboxamido, $C_1$-$C_4$alkylaminocarbonyl, amino, mercapto, $C_1$-$C_4$alkoxy, $C_3$-$C_8$cycloalkyl, imidazolyl, indolyl, pyrrolidinyl, hydroxypyrrolidinyl, halogen, phenyl or phenoxy each of which is unsubstituted or monosubstituted or polysubstituted by hydroxyl, $C_1$-$C_4$alkyl, halogen, nitro or trifluoromethyl, $C_3$-$C_{81}$alkyl interrupted one or more times by -O- or ($C_3$-$C_{81}$alkoxy)carbonyl interrupted one or more times by -O-;
$R^2$ and $R^3$ independently of one another denote hydrogen, $R^1$ or, together with the nitrogen to which they are bonded, a 5- or 6-membered, saturated heterocyclic ring which can additionally contain -O- or $-NR^5-$,
or $R^2$ denotes hydrogen and $R^3$ denotes a radical of the formula

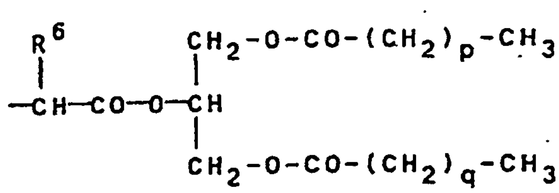

or

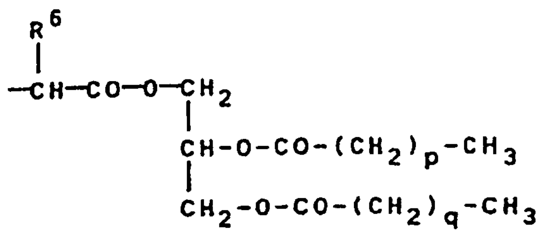

$R^4$ denotes the radical of an amino acid or of a dipeptide, in which the peptide bond can also be reduced to $-NH-CH_2-$,
$R^5$ denotes hydrogen, $C_1$-$C_4$alkyl, phenyl, $C_1$-$C_4$alkylphenyl, $C_1$-$C_4$alkoxyphenyl,
$R^6$ denotes hydrogen, $C_1$-$C_4$alkyl, phenyl, phenyl ($C_1$-$C_4$)alkyl,
p and q independently of one another denote a number from 1 to 20,
and their physiologically tolerable salts.

2. Compounds according to Claim 1, **characterised in that** the radicals representing X contain 1,4- or 1,3-phenylene.

3. Compounds according to Claim 1 and/or 2, **characterised in that** the radicals representing X contain p-piperidinyl or 3-pyrrolidinyl.

4. Compounds according to one or more of Claims 1 to 3, **characterised in that** R denotes $-N(R^2)R^3$ or $-NHR^4$.

5. Compounds according to one or more of Claims 1 to 4, **characterised in that** R denotes $-NHR^4$, where $R^4$ represents the valine, phenylalanine or phenylglycine radical, which is formally formed by removal of the amino group from valine, phenylalanine or phenylglycine.

6. Process for the preparation of aspartic acid derivatives of one or more of Claims 1 to 5, **characterised in that**

a) a compound of the general formula II

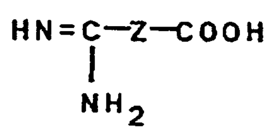

(II)

in which
Z denotes

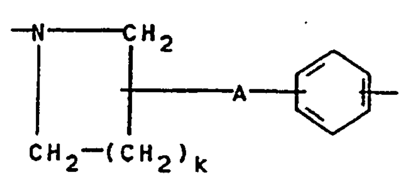

and A and k have the meanings mentioned in Claims 1 to 5, is reacted with a compound of the general formula III

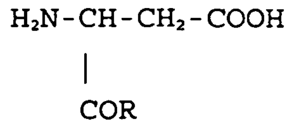 (III)

in which R has the meaning mentioned in Claims 1 to 5, or

b) a compound of the general formula Ia

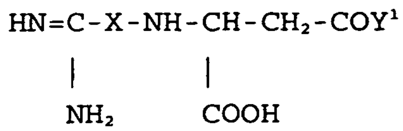 (Ia)

in which X has the meaning already mentioned at the beginning and $Y^1$ denotes a protective group, is reacted with a compound of the formula IV

HR (IV)

in which R has the meaning already mentioned at the beginning, and then the protective group is removed, and **in that** the compound of the formula I is optionally converted into a physiologically tolerable salt.

**7.** Process according to Claim 6, **characterised in that** amino groups, amidino groups, guanyl groups, mercapto groups and/or carboxyl groups in the starting materials employed carry protective groups which are optionally removed in a manner known per se after reaction is complete.

**8.** Compound of one or more of Claims 1 to 5 as inhibitor of platelet aggregation, metastasis of carcinoma cells or osteoclast binding to the bone surface.

**9.** Pharmaceutical preparation, **characterised in that** it contains one or more compounds of the general formula I of one or more of Claims 1 to 5 or a physiologically tolerable salt thereof as active compound together with pharmaceutically acceptable excipients and additives and, if desired, additionally one or more other pharmacological active compounds.

**10.** Process for the production of a pharmaceutical preparation containing one or more compounds of the general formula I of one or more of Claims 1 to 5 or a physiologically tolerable salt thereof, **characterised in that** these are brought into a suitable administration form together with pharmaceutically acceptable excipients and additives and, if desired, additionally one or more other pharmacological active compounds.